# EUROPÄISCHE PATENTSCHRIFT

(11) **EP 0 575 970 B1**
(45) Veröffentlichungstag und Bekanntmachung des Hinweises auf die Patenterteilung: **08.09.1999**
(21) Anmeldenummer: 93109998.0
(22) Anmeldetag: 23.06.1993
(51) Int. Cl.: A61M 1/16, A61J 1/10, A61M 39/00

(54) **Konzentrat enthaltender Beutel**
Bag containing a concentrate
Pochette contenant un concentré

(30) Priorität: 26.06.1992 DE 4220647 U; 05.02.1993 DE 4303372
(43) Veröffentlichungstag der Anmeldung: 29.12.1993
(73) Patentinhaber: Fresenius Medical Care Deutschland GmbH, 61350 Bad Homburg v.d.H. (DE)
(72) Erfinder: Polaschegg, Hans-Dietrich, Dr., D-6370 Oberursel 4 (DE); Pieper, Walter, D-6364 Florstadt 1 (DE); Weber, Daniel, Dr., F-47220 Astaffort (FR)
(74) Vertreter: Luderschmidt, Schüler & Partner GbR

(56) Entgegenhaltungen:
- EP-A- 0 198 407
- WO-A-83/00812
- DE-A- 3 844 174
- DE-U- 8 633 262
- DE-U- 8 813 659
- DE-U- 9 217 989
- US-A- 3 852 196
- US-A- 4 132 644
- US-A- 4 610 782

## Beschreibung

Die Erfindung betrifft einen Beutel, der ein flüssiges oder festes Konzentrat zur Herstellung einer Dialysierflüssigkeit enthält und eine Öffnung aufweist, an der ein die Öffnung dicht umgebender Steckerteil vorgesehen ist, der mit dem Behälterinneren in Strömungsverbindung steht.

Üblicherweise werden bei den heute eingesetzten Hämodialysemaschinen die für die Herstellung der Dialysierflüssigkeit eingesetzten Konzentrate werksseitig hergestellt und mit Kanistern zum Verbraucher befördert. Sowohl die Herstellung als auch die Beförderung derartiger mit flüssigem Konzentrat gefüllter Kanister ist teuer, da praktisch überwiegend nur Wasser vom Hersteller zum Patienten befördert wird und darüber hinaus die leeren Kanister ein Umweltproblem darstellen. Besonders problematisch ist die Lagerung und Beförderung von Kanistern, die mit einem Bicarbonat-Konzentrat gefüllt sind, da die Kanister durch die Ausgasung von Kohlendioxid aufgebläht werden und somit unter Überdruck stehen. Insofern existiert bei derart aufgeblähten Behältnissen ein Berstproblem bei unsachgemäßem Umgang mit den Kanistern.

Deshalb wurden bettseitige Systeme vorgeschlagen, mit denen entweder fertige Dialysierflüssigkeiten zur Verfügung gestellt oder pulverförmige Konzentrate, insbesondere Bicarbonat-Konzentrate, mit Wasser vermischt werden.

Zu dem ersten System gehören die sehr lang bekannten zentralen Dialysierflüssigkeit-Versorgungsanlagen, die insbesondere in Ringleitungen in der Klinik für mehrere Dialysemaschinen Dialysierflüssigkeit zur Verfügung stellen.

Andererseits werden auch pulverförmige Konzentrate in Behältern vorgelegt, die über ein Leitungssystem mit der Dialysemaschine einerseits und über eine Wasserleitung mit einer Wasserquelle andererseits verbunden sind. Mit einem derartigen System wird on-line frisches Bicarbonat-Konzentrat zur Verfügung gestellt, das unmittelbar ohne wesentliche Ausgasung von CO₂ der Dialysemaschine zur Verfügung gestellt werden kann. Ein solches System ist beispielsweise in den EP-A1-0 278 100 und EP-A1-0 443 324 beschrieben.

Die DE-U-8813659 beschreibt eine Vorrichtung zur Abgabe von flüssigen Präparaten, die zwei an einem gemeinsamen Entnahmeschlauch angeschlossene Beutel umfaßt. Die Beutel sind zwar mit Steckverbindern zum Anschluß der zugehörigen Zweigleitungen versehen, die bekannten Steckverbinder erlauben aber nur die Erzeugung eines Strömungspfades von dem Beutelinneren zu dem Abnehmer. Die bekannten Beutel sind nicht dazu bestimmt, mit einem Konzentrat befüllt zu werden, das mit einer dem Beutel zuzuführenden Flüssigkeit verdünnt werden soll.

Die WO-A-8300812 beschreibt zwar eine Steckverbindung mit zwei Strömungspfaden. Diese Steckverbindung ist aber nicht Bestandteil eines Beutels zur Aufnahme von Konzentrat für die Herstellung von Dialyseflüssigkeit. Über den einen Verbindungspfad des bekannten Steckverbinders fließt Dialyseflüssigkeit, während durch den anderen Flüssigkeitspfad nach dem Wechsel des Vorratsbehälters ein Desinfektionsmittel geleitet wird, um die in der Kupplung vorhandenen Hohlräume auszuspülen. Die Einlaß- bzw. Auslaßanschlüsse des bekannten Steckverbinders stehen nicht mit einem gemeinsamen Reservoir in Verbindung.

Die US-A-3 852 196 beschreibt eine Filtereinheit, die aus einem das Filtermaterial aufnehmenden Behälter besteht, der über einen doppellumig ausgebildeten Steckerteil verlügt. Der Steckerteil weist einen ersten Strömungspfad auf, über den ein Fluid in das Behälterinnere strömt und einen zweiten Pfad, über den das zuströmende Fluid aus dem Behälter abfließt. Die Filtereinheit kann an ein komplementär ausgebildetes Steckerteil zur Herstellung der Strömungsverbindung angeschlossen werden.

Die US-A-4 610 782 beschreibt ein Hämodialysesystem mit einem als Glaskolben ausgebildeten Behälter, der mit Dialysierflüssigkeit befüllt wird. Der Behälter ist weder mit Konzentrat zur Herstellung der Dialysierflüssigkeit befüllt, noch verfügt dieser über ein doppellumiges Steckerteil.

Die DE-A-38 44 174 offenbart eine Anlage zur Herstellung von Konzentrat durch Mischung von Flüssigkeit mit löslichem Stoff. Der Mischbehälter weist zwar einen kombinierten Anschlußstutzen auf, der als Rohr-im-Rohr-System ausgeführt ist. Dieser dient aber nicht zur Herstellung einer Verbindung zur Dialysemaschine, sondern zum Anschluß einer Pumpe, die Bestandteil der Anlage zur Herstellung des Konzentrates ist.

Der Erfindung liegt die Aufgabe zugrunde, einen flüssiges oder festes Konzentrat zur Herstellung einer Dialysierflüssigkeit enthaltenden Beutel zu schaffen, der ein verhältnismäßig geringes Volumen und Gewicht aufweist und die Versorgung einer Dialysemaschine mit Dialysierflüssigkeit vereinfacht.

Die Lösung dieser Aufgabe erfolgt erfindungsgemäß mit den Merkmalen des Patentanspruchs 1.

Der erfindungsgemäße Beutel besteht üblicherweise aus flexiblem Material, wie beispielsweise PVC, PVP oder Polyethylen. Die unmittelbar im Beutelkörper, d.h., in der Wand des Beutels angeordnete Steckerverbindung ersetzt die bisher übliche Schlauchverbindung zwichen Dialysemaschine und Konzentratbehälter. Es werden somit in vorteilhafter Weise Leitungswege verkürzt. Der Beutel ist durch die Steckverbindung einfach zu wechseln und sicher an der Dialysemaschine zu befestigen.

Es ist als besonderer Vorteil des erfindungsgemäßen Beutels anzusehen, daß der Konzentratbehälter über einen einzigen Anschluß, der das Zuströmen von Wasser und das Abführung von verdünntem Konzentrat ermöglicht, mit der Dialysemaschine verbunden ist.

Die Sättigung des zuströmenden Wassers mit zu verdünnendem Konzentrat wird gefördert, wenn die zuströmende Flüssigkeit durch einen Schlauch in das Innere des Beutelkörpers geleitet wird. Vorzugsweise wird die verdünnte Konzentratlösung über einen in das Beutelinnere hineinragenden Schlauch, der an seinem Ansaugende mit einem Filter versehen ist, der Dialysemaschine zugeführt. Die Strömungspfade für die zu- und abströmenden Lösungen können in der Steckverbindung parallel oder konzentrisch angeordnet sein. Bei konzentrischer Anordnung der Strömungspfade in der Steckeranordnung ist es vorteilhaft, wenn Einlaß und Auslaß in den Beutelkörper am beutelseitigen Steckerteil parallel angeordnet sind.

In bevorzugter Ausführungsform weist der erfindungsgemäße Beutel an seinem Steckerteil eine Kodierung auf, die jeweils einen bestimmten Beutelinhalt kennzeichnet. So wird sichergestellt, daß stets der richtige Konzentratbehälter an die richtige Flüssigkeitszuleitung der Dialysiermaschine angeschlossen wird. Um während des Transportes Beschädigungen oder Verschmutzungen des beutelseitigen Steckerteils zu vermeiden, wird dieses bevorzugt bis zur Benutzung mit einer Verschlußkappe abgedeckt.

Bei dem doppellumigen Konzentrat-Stecker wird das der Steckerverbindung und dem Beutel zugeführte Wasser zunächst in festes oder flüssiges Konzentrat, beispielsweise Bicarbonatpulver, geleitet, und das gelöste Konzentrat, beispielsweise gesättigte Bicarbonatlösung, wird anschließend durch die Steckerverbindung zurück- und der Dialysemaschine zugeleitet.

Im folgenden wird die Erfindung anhand von Ausführungsbeispielen in Verbindung mit den beigefügten Zeichnungen beschrieben.

Dabei zeigen:
- Fig. 1: eine schematische Schnittansicht eines Ausführungsbeispiels eines geräteseitigen, zweiten Steckerteils zum Anschluß des erfindungsgemäßen Beutels mit einem beutelseitigen ersten Steckerteil, wobei eine Schließstellung des Ventilelements gezeigt ist,
- Fig. 2: eine Seiten-Schnittansicht der Steckerverbindung gemäß Figur 1, wobei der mit Bicarbonatpulver gefüllte erfindungsgemäße Beutel mit dem doppellumigem Steckerteil angeschlossen ist,
- Fig. 3: eine schematische Schnittansicht eines Bicarbonat-Beutelsteckers, ähnlich dem in Fig. 2 gezeigten Stecker und
- Fig. 4: eine weitere Ausführungsform des zweiten Steckerteils zum Anschluß des erfindungsgemäßen Beutels in der Schließstellung im Längsschnitt,
- Fig. 5: eine Ausführungsform des an den zweiten Steckerteil von Figur 4 angeschlossenen ersten Steckerteils des erfindungsgemäßen Beutels im Längsschnitt,
- Fig. 6: die beweglichen Elemente des in Figur 4 und 5 dargestellten zweiten Steckerteils im Längsschnitt (90° gedreht, bezogen auf Figur 5),
- Fig. 7: eine weitere Ausführungsform des zweiten Steckerteils zum Anschluß des erfindungsgemäßen Beutels im Längsschnitt und
- Fig. 8: eine Ausführungsform des an den zweiten Steckerteil von Figur 7 angeschlossenen ersten Steckerteils des erfindungsgemäßen Beutels.

Obwohl der Vollständigkeit halber und des besseren Verständnisses wegen nachfolgend auch das geräteseitige Steckerteil bzw. die aus dem geräteseitigen und beutelseitigen Steckerteil bestehende Steckverbindung beschrieben ist, bezieh sich die Erfindung aber nur auf den Beutel mit dem beutelseitigen Steckerteil.

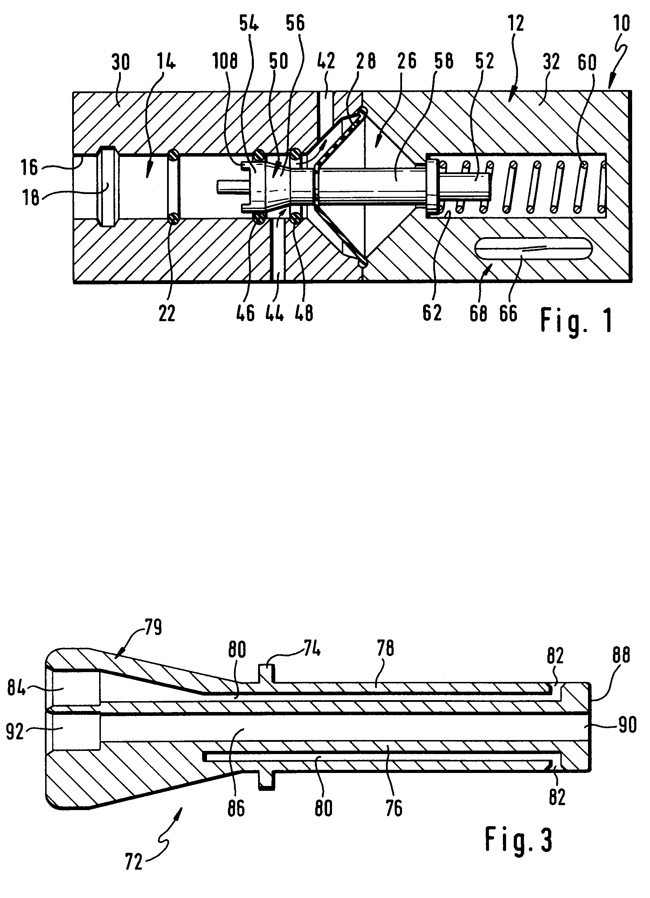

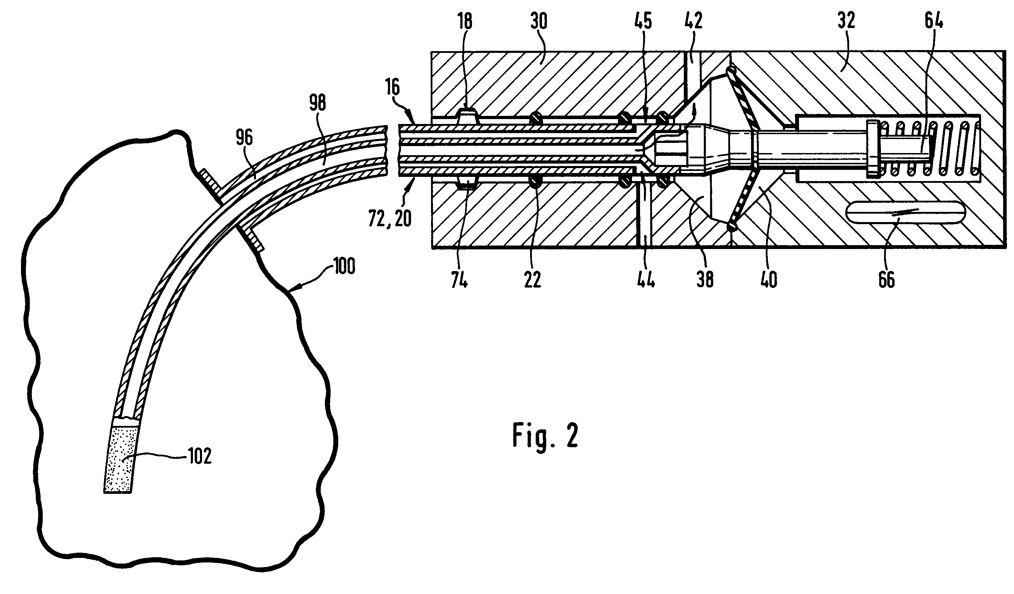

Nachfolgend wird die Ausführungsform einer Steckerverbindung gemäß Figur 1 und 2 beschrieben. Die Steckerverbindung besteht aus einem ersten und zweiten Steckerteil, wobei das erste Steckerteil mit dem Konzentratbeutel verbunden ist (beutelseitiges Steckerteil); das zweite Steckerteil ist an der Dialysemaschine angeordnet (geräteseitiges Steckerteil oder Kupplungsbuchse).

Die Steckerverbindung weist zunächst ein an der Dialysemaschine angeordnetes, geräteseitiges zweites Steckerteil auf, nachfolgend auch als Kupplungsbuchse 10 bezeichnet. Die Kupplungsbuchse 10 weist einen Buchsenkörper 12 auf, der mit einer im wesentlichen zylindrischen, sich über eine Teillänge des Buchsenkörpers 12 erstreckenden, im wesentlichen zylindrischen Ausnehmung 14 versehen ist, die in Teilbereichen unterschiedliche Durchmesser aufweist.

Nach ihrem freien Einführungsende 16, das der Eingangsseite zugewandt ist, weist die Ausnehmung 14 eine Nut 18 auf, welche einen Teil von Rastmitteln bildet, um einen beutelseitigen Stecker 20 - wie nachfolgend erläutert - zu fixieren. Weiterhin ist in der Ausnehmung 14 innenliegend vorteilhafterweise ein Dichtring 22 vorgesehen, um den beutelseitigen Stecker im konnektierten Zustand zur Umgebung hin abzudichten.

Im mittigen Teil der Ausnehmung 14 ist mit einem größeren Durchmesser eine Ventilkammer 26 vorgesehen, in welcher quer zur Hauptachse des Buchsenkörpers 12 eine elastische Wand 28 eingespannt ist. Hierzu ist der Buchsenkörper 12 in zwei Buchsenteile 30 und 32 geteilt, so daß die Wand 28 in die Trennfuge 34 mittels der Schrauben 36 eingespannt werden kann. Die Wand 28 teilt dabei die Ventilkammer 26 in eine linke Kammerhälfte 38 und eine rechte Kammerhälfte 40, die in ihrem Volumen aufgrund der Verschiebung der Wand 28 variabel sind.

Von der linken Kammerhälfte 38 geht ein Auslaßkanal 42 ab, welcher mit dem Dialysierflüssigkeitssystem einer nicht dargestellten Dialysemaschine verbunden werden kann. Andererseits ist diese Ventilkammer-Hälfte 38 über die zylindrische Ausnehmung 14 mit einem Einlaßkanal 44 in Strömungsverbindung, der im angrenzenden Bereich 45 zur Ventilkammer-Hälfte 38 in die Ausnehmung 14 mündet.

Wie aus Figur 1 und 2 ersichtlich ist, ist beiderseits des Einlaßkanals 44 jeweils eine ringförmige Dichtungsanordnung 46, 48 in Form eines O-Rings in der Ausnehmung 14 vorgesehen.

Desweiteren ist in der Ausnehmung 14 ein Ventilelement 50 angeordnet, welches rückwärtig einen zylindrischen Endbereich 52 aufweist, wobei das Element 50 und der Endbereich 52 durch die flexible Wand 28 voneinander getrennt sind.

Das Ventilelement 50 selbst weist einen im wesentlichen zylinderförmigen Frontbereich 54 und daran rückwärts anschließend einen sich verjüngenden Kegelbereich 56 auf. An den Kegelbereich 56 schließt sich der den zylindrischen Endbereich 52 bildende Schaft 58 an, gegen dessen Ende ein elastisches Element 60 in Form einer Feder anliegt, welches in dem Endbereich 62 der Ausnehmung 14 angeordnet ist. An dem Ende des Schafts 58 ist ein Magnet 64 befestigt, welcher mit einem am Teil 32 angeordneten Reed-Schalter 66 zusammenwirkt, wobei der Magnet und der Reed-Schalter 66 einen Sensor 68 bilden, mit dem der Kupplungsvorgang überwacht werden kann.

In Figur 1 ist ein Betriebszustand gezeigt, bei welchem sich kein beutelseitiger Stecker 20 in der Ausnehmung 14 befindet. Durch die Feder 60 wird das Ventilelement 50 so weiter nach links vorgespannt, wie dies durch einen den Schaft 58 umgebenden Anschlag möglich ist, der sich an der rechten Kammerhälfte 40 abstützt. Hierdurch gelangt der zylindrische Frontbereich 54 des Ventilelements 50 in dichtenden Eingriff zur linken ringförmigen Dichtungsanordnung 46, sperrt somit den Einlaßkanal 44 gegenüber der Umgebung ab. Diese Stellung wird als Schließstellung bezeichnet.

Andererseits entsteht im Bereich des Kanals 44 durch die kegelförmige Anordnung des Kegelbereichs 56 ein Ringraum im Bereich des Einlaßkanals 44, so daß durch diesen Ringraum und die linke Ventilkammer-Hälfte 38 hindurch eine Strömungsverbindung zum Auslaßkanal 42 geschaffen wird. Somit ist eine Durchspülung der Kupplungsbuchse 10 und damit der Dialysemaschine mit Wasser oder Desinfektionslösung möglich.

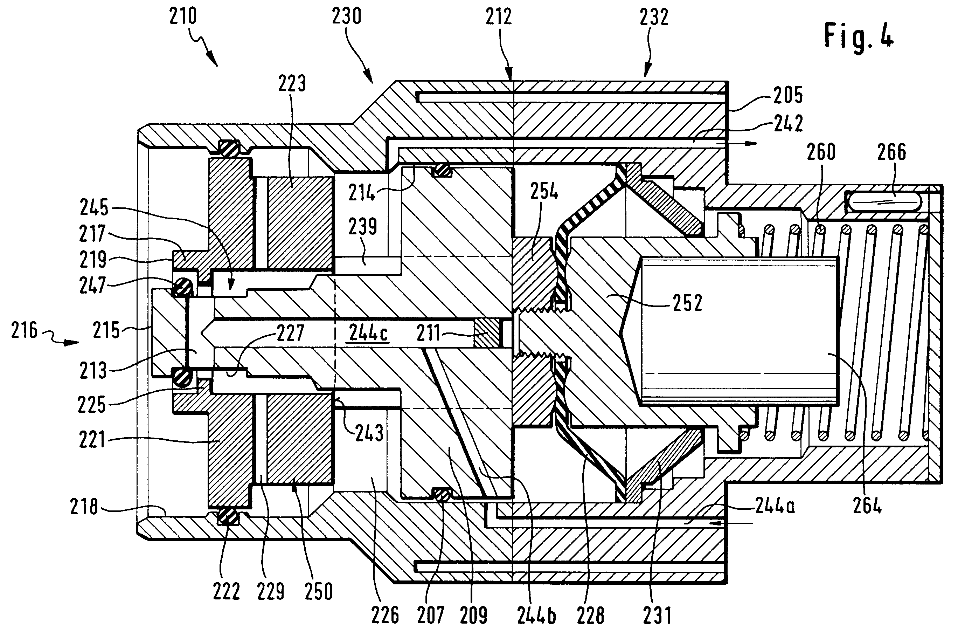

In Figur 2 ist ein Betriebszustand gezeigt, bei welchem der beutelseitige Stecker 20 in die Ausnehmung 14 eingeschoben ist, wobei Rastmittel 74, die auf dem Stecker 72 angeordnet sind, mit der Rastausnehmung 18 im Eingriff stehen. Gemäß der in Figur 3 gezeigten Ausführungsform des beutelseitigen Steckers ist der beutelseitige Stecker 72 doppellumig ausgebildet und umfaßt ein inneres Rohr 76 sowie ein äußeres Rohr 78, die in ein rückwärtiges Griffteil 79 münden und zwischen welchen ein ringförmiger Kanal 80 gebildet ist. Dieser ringförmige Kanal 80 weist eine vordere, die Außenoberfläche des äußeren Rohrs 78 durchbrechende Ausnehmung 82 auf, die vorteilhafterweise ringförmig das der Kupplungsbuchse 12 zugewandte Ende des ersten Steckerteils 72 umgibt. Im Griffteil 79 mündet der Kanal 80 in eine erste Zuführungsöffnung 84.

Das innere Rohr 76 durchsetzt ein zentraler Kanal 86, der aus dem Frontende 88, das dem Buchsenkörper 12 zugewandt ist, in der Öffnung 90 austritt und im Griffteil 79 in die Öffnung 92 mündet.

Im konnektierten Zustand, wie dies in Figur 2 gezeigt ist, kann Wasser aus der Leitung 44 durch die Öffnung 82, den Kanal 80, die Öffnung 84 im Griffteil 79 (vgl. Fig. 3) und einen an den Griffteil 79 anschließenden Kanal 96 des beutelseitigen Steckers in den Beutel 100 fließen und löst dort das Bicarbonat auf. Aufgrund des geschlossenen Beutels 100 fließt die Bicarbonatlösung durch ein an einem in das Beutelinnere ragenden Schlauch angeordnetes Filter 102 und einen an den Griffteil 79 anschließenden Kanal 98 des Steckers 72, gelangt durch die Öffnung 92 im Griffteil 79 in den Kanal 86 und schließlich durch die Öffnung 90 in die linke Kammerhälfte 38 (vgl. Fig. 2), von der es in den Auslaßkanal 42 fließt. Insofern kommt es also zu einem Kreislauf durch die erfindungsgemäße Konnektoranordnung, wobei es zu einer Vermischung von Wasser und Bicarbonat und damit zu einer Herstellung eines Bicarbonatkonzentrats kommt.

Das Frontende 88, das ringförmig die Öffnung 90 umgibt, ist - wie sich dies besonders aus Figur 1 und 3 ergibt - mit mehreren voneinander getrennten Ansätzen 108 in Eingriff, die auf dem Ventilelement 50 kreisförmig angeordnet sind. Zwischen den Ansätzen 108 kann also Flüssigkeit bei gekuppeltem Stecker am Ventilelement 50 vorbei in die linke Kammerhälfte 38 fließen.

Die Figuren 1, 2 und 3 zeigen weiterhin, daß der Stecker 72 im konnektierten Zustand derart mit dem Einlaßkanal 44 über die Öffnung 82 in Strömungsverbindung steht, daß beiderseits der Öffnung 82 die beiden ringförmigen Dichtungsanordnungen 46 und 48 gegen das äußere Rohr 78 abdichten. Andererseits liegt die Frontöffnung 90 des Steckers 72 jenseits der innenliegenden ringförmigen Dichtungsanordnung 48 auf der Seite der linken Kammerhälfte 38 und kann somit nicht mit der Öffnung 82 unmittelbar in Strömungsverbindung gebracht werden (nur über den Beutel 100).

Figur 2 zeigt weiterhin schematisch, daß der Magnet 64 im Kupplungszustand den Reed-Schalter 66 und somit den Senor 68 betätigt, so daß die betriebsfertig montierte Stellung des Steckers 72 festgestellt werden kann.

Gemäß eines weiteren Ausführungsbeispiels der Erfindung, dargestellt in den Fig. 4 bis 6 soll an dem zweiten Steckerteil mittels eines korrespondierenden ersten Steckerteils ein flexibler Beutel mit Bicarbonatpulver anschließbar sein, wobei durch die Steckerverbindung Flüssigkeit zum Beutel zu- und Bicarbonatlösung abgeführt werden soll. Der Aufbau des Buchsenkörpers und der Ventilkammer, insbesondere der rechten Hälfte der Ventilkammer mit den Steuerelementen gleicht der vorstehend beschriebenen Ausführungsform der Erfindung. Im folgenden werden daher im wesentlichen die Unterschiede im ersten Steckerteil sowie im Eingangsteil des zweiten Steckerteils zu den Ausführungsformen gemäß der Fig. 1 bis 3 geschildert.

Das Eingangsteil 230 des Buchsenkörpers 212 ist dem aufzusteckenden ersten Steckerteil 220 des Bicarbonatbeutels 200 zugewandt. Diese Seite wird nachfolgend als Eingangsseite 216 bezeichnet. Das Ausgangsteil 232 des Buchsenkörpers 212 schließt einerseits am Eingangsteil 230 des Buchsenkörpers an, in die gegenüberliegende Stirnfläche 205, nachfolgend Ausgangsseite genannt, münden die Kanäle 244a und 242.

In das Eingangsteil 230 sind eine Dichtbuchse 209 und eine Ventilbuchse 250 eingesetzt. Die Dichtbuchse ist ortsfest eingepaßt. Sie weist eine zentrale, sich in Längsrichtung erstreckende Bohrung 244c als Fortsetzung des Zuflußkanals 244a,b auf. Von der zentralen Bohrung 244c erstreckt sich ein verbindender Kanal 244b zum Kanalabschnitt 244a, wobei der Kanalabschnitt 244a in die Wandung des Buchsenkörpers 212 eingearbeitet ist. Es wird also eine durchgehende Flüssigkeitsverbindung von der Stirnfläche 205 bis in die zentrale Bohrung 244c der Dichtbuchse 209 geschaffen. Der zwischen dem Kanalabschnitt 244b und dem der Ausgangsseite 205 zugewandten Ende der Dichtbohrung 209 liegende Abschnitt der Zentralbohrung 244c ist durch einen Dichtstopfen 211 verschlossen. Das der Eingangsseite 216 zugewandte Ende der zentralen Bohrung mündet in eine Querbohrung 213, die sich parallel zur Eingangsseite 216 erstreckt. Die stirnwand 215 schließt die Dichtbuchse 209 zur Eingangsseite hin ab.

Die Dichtbuchse 209 ist an ihrem der Ausgangsseite 205 zugewandten Ende so ausgeformt, daß sie sich abdichtend in die Bohrung 214 einfügt. Zur sicheren Abdichtung ist zwischen Buchse und Buchsenkörper im Buchsenkörper 212 ein O-Ring 207 eingesetzt. Zwischen der Einmündung des Kanals 242 für die Flüssigkeitsableitung in die Bohrung 214 und der Eingangsseite 216 ist der Durchmesser der Dichtbuchse 209 wesentlich reduziert. Der zwischen der Dichtbuchse und dem Eingangsteil 230 des Buchsenkörpers in der Ventilkammer 226 entstehende Zwischenraum wird von dem beweglich angeordneten Ventilelement, einer zylinderförmigen Ventilbuchse 250, teilweise abdichtend ausgefüllt.

Die Ventilbuchse 250 weist ebenfalls Abschnitte mit verschiedenen Durchmessern auf. Ein erster kreisringförmiger Abschnitt 217 erstreckt sich von der Eingangsseite in die Bohrung 214 hinein. Dieser Abschnitt 217 weist einen deutlich kleineren Durchmesser auf als die Bohrung 214. Die der Eingangsseite zugewandte Stirnfläche des Abschnitts 217 dient als Anschlagsfläche 219 für das beutelseitige Steckerteil. An den ersten kreisringförmigen Abschnitt 217 schließt sich an dem der Anschlagsfläche 219 gegenüberliegenden Ende ein zweiter kreisringförmiger Abschnitt 221 an, der abdichtend in die Bohrung 214 eingefügt ist. Ein zwischen diesem Abschnitt 221 und dem Buchsenkörper 230 in den Buchsenkörper eingefügter O-Ring 222 dichtet das geräteseitige Steckerteil 210 sicher nach außen ab. Ein dritter kreisringförmiger Abschnitt 223 bildet den zum Ausgangsteil 232 hin gerichteten Abschluß der Ventilbuchse 250. Der Durchmesser dieses Abschnitts 223 ist so gewählt, daß er sich mit Spiel in eine korrespondierende Verengung der Bohrung 214 fügt. Anders als bei der in Fig. 1 und 2 beschriebenen Ausführungsform ist das Ventilelement 250 hier nicht einstückig zur Anlage an die Feder 260 ausgebildet. Hier wird die Federkraft vielmehr über eine Magnetbuchse 252 und einen Stößel 254 auf das Ventilelement 250 übertragen. Die Ventilbuchse 250 sitzt mit einem ausreichenden Spiel auf der Dichtbuchse 209, so daß sie ohne weiteres verschiebbar ist, und daß außerdem genügend Raum für den Flüssigkeitsdurchtritt verbleibt. Lediglich im Bereich des ersten 217 und/oder zweiten 221 kreisförmigen Abschnitts der Ventilbuchse 250 erstreckt sich eine Verstärkung 225 der Ventilbuchse 250 in enge Anlage an die Dichtbuchse 209. Im Bereich der Verstärkung 225 weist die Dichtbuchse 209 eine Ausnehmung 227 auf, in der ein beweglicher O-Ring 247 angeordnet ist, der in der Ausnehmung 227 durch die Verstärkung 225 geführt wird. Die Querbohrung 213 mündet im Bereich der Ausnehmung 227. Der Bereich 245, in dem das Ventilelement 250 bewegbar ist, erstreckt sich so weit, daß in der Schließstellung eine sichere Abdichtung des zweiten Steckerteils nach außen gewährleistet ist, und daß bei aufgestecktem Beutel die Strömungspfade für die Flüssigkeitszu- bzw. ableitung sicher getrennt sind. Die Ventilbuchse 250 weist im dritten kreisringförmigen Abschnitt 223 eine radiale, parallel zur Eingangsseite verlaufende Bohrung 229 auf.

Durch Verschieben der Ventilbuchse 250 werden die im Ausgangsteil 232 des Buchsenkörpers angeordneten Steuerbauteile betätigt. Das Verschieben wird entweder durch Anschließen des Bicarbonatbeutels oder durch Aufstecken einer Verschlußkappe (nicht dargestellt), in der die beiden Strömungspfade (Zu- bzw. Ablauf) kurzgeschlossen werden, bewirkt. Die Verschlußkappe wird aufgesteckt, um ein vollständiges Sterilisieren des zweiten Steckerteils 212 zu ermöglichen.

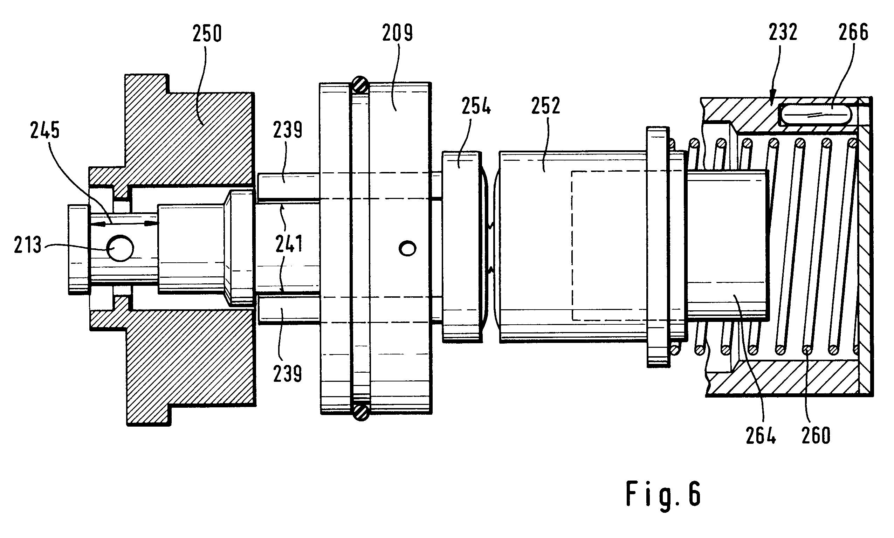

Durch das Einfügen der Dichtbuchse 209 in die Ventilkammer ist das bewegliche Ventilelement 250 nicht einstückig mit den Steuerbauteilen der rechten Ventilkammerhälfte verbunden. Die Betätigung erfolgt nunmehr über den mit der nunmehr Magnetbuchse 252 verbundenen Stößel 254. Der Stößel ist mit der Magnetbuchse verschraubt, und im Bereich der Verschraubung wird dabei gleichzeitig die elastische Wand 228 in der Mitte der Bohrung 214 fixiert. Der Stößel 254 weist im Bereich der Verschraubung mit der Magnetbuchse 252 den gleichen Außendurchmesser auf wie diese. Ein zweiter Abschnitt des Stößels mit geringerem Durchmesser erstreckt, wie besonders aus Fig. 6 ersichtlich, aus dem Abschnitt 232 des Buchsenkörpers in den Eingangsabschnitt 230. Dieser zweite Abschnitt des Stößels 254 besteht aus zwei Kreisbogensegmenten, die sich als Gabelarme 239 durch entsprechende Ausnehmungen in der Dichtbuchse 209 hindurch erstrecken. Die Gabelarme 239 werden in Aussparungen 241 der Dichtbuchse geführt, doch ragen die Gabelarme über den Durchmesser der Dichtbuchse 209 hinaus. Sie überlappen im Bereich einer Anlagefläche 243 mit der der Ausgangsseite 205 zugewandten Fläche der Ventilbuchse 250. Über die Anlagefläche 243 wird die Verschiebebewegung von der Ventilbuchse 250 bis auf den Magneten 264 übertragen.

Nachstehend wird die Funktionsweise des beschriebenen zweiten Steckerteils 210 in der Schließstellung (ohne Beutel) und bei angeschlossenem Bicarbonatbeutel bzw. aufgesteckter Verschlußkappe erläutert.

In Fig. 4 wird das zweite Steckerteil 210 ohne angehängtem Bicarbonatbeutel bzw. ohne dem zum Sterilisieren des zweiten Steckerteils aufgesteckten Verschluß gezeigt. In dieser Stellung drückt die vorgespannte Druckfeder 260 die Magnetbuchse 252 gegen die Führungsbuchse 231. Da der Stößel 254 starr mit der Magnetbuchse 252 verbunden ist, erfährt er in dieser Stellung eine maximale Auslenkung in das Eingangsteil 230 des Buchsenkörpers. Damit wird über die Anlagefläche 243 auch die Ventilbuchse 250 maximal zur Eingangsseite hin ausgelenkt. In dieser Position wird der O-Ring 247 zwischen der Verstärkung 225 der Ventilbuchse 250 und der eingangsseitigen Begrenzung der Ausnehmung 227 der Dichtbuchse 209 fixiert. Der Flüssigkeitsdurchtritt zwischen der Dichtbuchse 209 und der Ventilbuchse 250 ist somit wirksam nach außen abgedichtet. Gleichzeitig liegt der zweite Abschnitt 221 der Ventilbuchse 250 abdichtend an dem in den Buchsenkörper 230 eingefügten O-Ring 222 an. Somit ist auch ein Flüssigkeitsdurchtritt zwischen Ventilbuchse 250 und Buchsenkörper 230 ausgeschlossen.

In dieser Stellung kann das zweite Steckerteil 210 gespült werden. Wie am Eingang des Flüssigkeitszufuhrkanals 244a durch die Pfeilrichtung angezeigt, strömt Wasser oder Desinfektionslösung durch den Kanal 244 in die zentrale Bohrung 244c und von dort tritt, da das andere Ende der zentralen Bohrung 244c durch den Dichtstopfen 211 verschlossen ist, die Flüssigkeit über die Querbohrung 213 in den Raum zwischen Dichtbuchse und Ventilbuchse. Durch die radiale Bohrung 229 gelangt die Flüssigkeit ebenfalls in den Raum zwischen Ventilbuchse und Buchsenkörper. Dadurch ist gewährleistet, daß keine Toträume entstehen, und daß das zweite Steckerteil 210 vollständig durchspült wird. Das Wasser bzw. die Desinfektionslösung werden schließlich durch den Kanal 242 für die Flüssigkeitsableitung abgeführt.

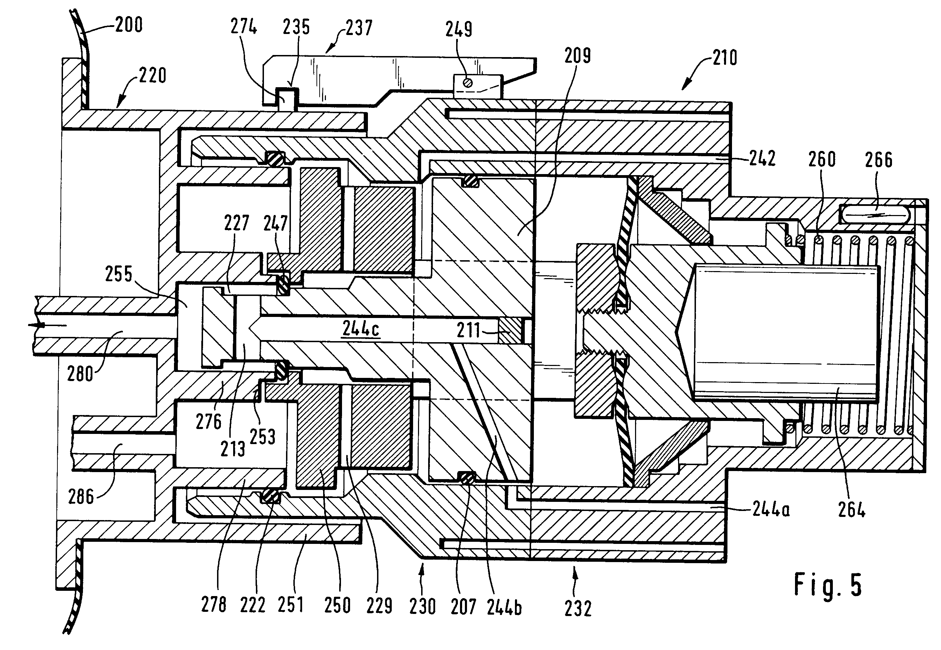

Aus Fig. 5 geht hervor, in welcher Stellung sich die beweglichen Bauteile des zweiten Steckerteils befinden, wenn ein Bicarbonatbeutel auf die Eingangsseite der Kupplungsbuchse aufgesteckt ist. Der Stecker 220 ist ohne zwischengeschaltete Schlauchleitung und unmittelbar mit dem Bicarbonatbeutel 200 verbunden. Zweites Steckerteil 210 und Stecker 220 werden über eine Rastsicherung 237 fest miteinander verbunden. Die Rastsicherung 237 ist über ein Lager 249 an der Kupplungsbuchse bewegbar befestigt und greift mit einer Rastöffnung 235 über eine Rastnase 274 des Steckers 220. An der dem Bicarbonatbeutel 200 abgewandten Seite weist der Stecker 220 drei konzentrische Ringwände auf. Die erste, äußere Ringwand 251 trägt die Rastnase 274 und umschließt einen Abschnitt des Buchsenkörpers 230. Diese äußere Ringwand 251 ist die am weitesten auskragende Ringwand.

Die in bezug auf die äußere Ringwand zurückversetzte Ringwand 278 steht im abdichtenden Eingriff mit der in den Buchsenkörper 230 eingefügten Ringdichtung 222. Die innere Ringwand 276 ist gegenüber der mittleren Ringwand 278 nochmals zurückversetzt. Bei aufgeschobenen Stecker liegt die dem zweiten Steckerteil 210 zugewandte Stirnfläche 253 der inneren Ringwand an der der Eingangsseite zugewandten Stirnfläche der Ventilbuchse 250 an. Somit wird beim Aufstecken des Steckers 220 durch das Anliegen der Stirnfläche 253 an die Ventilbuchse 250 letztere gegen den Druck der Feder 260 zur Ausgangsseite des zweiten Steckerteils 210 hin verschoben. Wenn der Stecker 220 in der Endstellung mit der Rastsicherung 274 an dem zweiten Steckerteil 210 verrastet ist, wird zunächst durch das Verschieben der Ventilbuchse 250 durch die Stirnfläche 253 der inneren Ringwand auch die Ringdichtung 247 von der eingangsseitigen Begrenzung der Ausnehmung 227 zur gegenüberliegenden, ausgangsseitigen Begrenzung der Ausnehmung 227 verschoben. Damit strömt das über den Zuführkanal 244a in die zentrale Bohrung 244c einströmende Wasser über die Querbohrung 213 aus dem zweiten Steckerteil 210 heraus in den von der inneren Ringwand 276 umschlossenen Zwischenraum 255 der durch den O-Ring 247 gegen das zweite Steckerteil 210 abgedichtet ist, und der mit einem Auslaß 280 versehen ist, durch das aus der Querbohrung 213 austretende Wasser in das Innere des Beutels 200 ausströmen kann. Die im Beutel 200 entstehende konzentrierte Bicarbonatlösung wird über eine Eingangsleitung 286, die an ihrem Eintrittsende mit einem hier nicht dargestellten Filter versehen ist, angesaugt. Die Eingangsleitung 286 mündet in den kreisförmigen Zwischenraum zwischen der inneren Ringwand 276 und der mittleren Ringwand 278. Ein Vermischen der Bicarbonatlösung mit dem zulaufenden Wasser ist ausgeschlossen, da der Übergang zwischen den beiden Hohlräumen durch die Ringdichtung 247 verschlossen ist. Die eintretende Bicarbonatlösung gelangt aus dem Hohlraum zwischen der inneren und der mittleren Ringwand in den Zwischenraum zwischen Ventilbuchse 250 und Buchsenkörper 230. Sie wird weiter durch die radiale Bohrung 229 in den Raum zwischen der Dichtbuchse 209 und der Ventilbuchse 250 geführt, um hier durch den Kanal 242 zur Ableitung der Bicarbonatlösung abgeführt zu werden. Es ist vorteilhaft, den Kanal 242 oberhalb des Zuführkanals 244 anzuordnen, damit sichergestellt ist, daß das etwa entstehende CO₂ zusammen mit der Bicarbonatlösung sicher abgeführt wird. Ein Austreten der Bicarbonatlösung aus dem zweiten Steckerteil 210 wird dadurch verhindert, daß die mittlere Ringwand 278 im abdichtenden Eingriff mit der Ringdichtung 222 steht. Die hier beschriebene zweite Ausführungsform der Erfindung ermöglichst eine besonders sichere Steckverbindung und gewährleistet über dies eine einfache und sichere Trennung der beiden Strömungspfade voneinander.

Durch das Verschieben des Magneten 264, d.h., durch Aufstecken eines Beutels wird wiederum ein Reed-Schalter 266 mit entsprechenden Steuerungsvorrichtungen (hier nicht dargestellt), bestätigt.

Wird zum Reinigen bzw. Sterilisieren des zweiten Steckerteils 210 eine Verschlußkappe auf die Eingangsseite des Buchsenkörpers 230 aufgesteckt, so ist diese Verschlußkappe in gleicher Weise aufgebaut wie der Stecker 220, mit dem Unterschied, daß die Leitungen 280 und 286 nicht jeweils offen in einem Beutel 200 enden, sondern vielmehr kurzgeschlossen sind. Mithin strömt das Wasser bzw. die Desinfektionslosung aus der Querbohrung 213 über die Kurzschlußleitung unmittelbar in den Zwischenraum zwischen innerer Ringwand 276 und mittlerer Ringwand 278 und zurück durch die Räume zwischen Buchsenkörper 230, Ventilbuchse 250 und Dichtbuchse 209 hin zum Abführkanal 242. Auf diese Weise werden sämtliche Flächen, die mit der Bicarbonatlösung in Berührung kommen, vollständig und gründlich gereinigt bzw. desinfiziert.

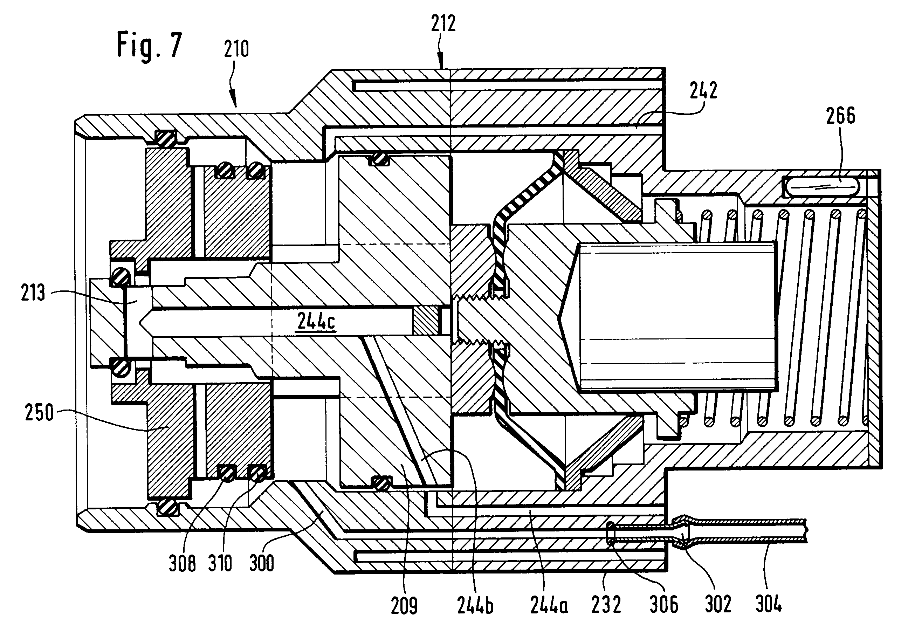

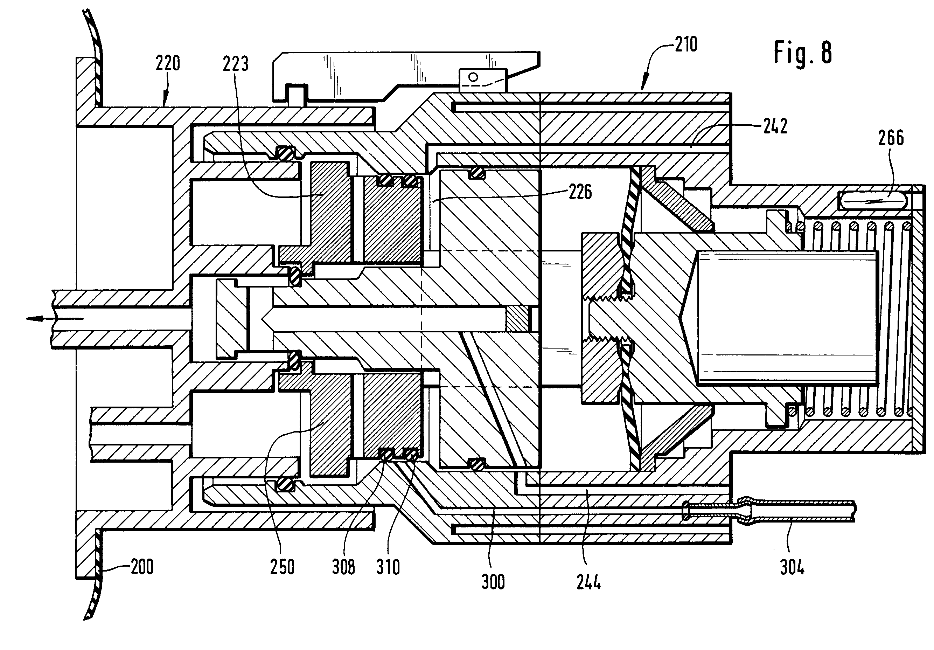

Eine weitere Ausführungsform der Steckerverbindung wird in den Fig. 7 und 8 dargestellt. Diese Ausführungsform trägt der Tatsache Rechnung, daß durch die Anordnung nicht nur Flüssigkeitskonzentrate aus Feststoffen erzeugt werden, sondern daß mittels ein und derselben Anordnung auch flüssige Konzentrate verdünnt werden können bwz. gegebenenfalls mehrere Konzentrate zu verdünnter Dialysierflüssigkeit oder Dialysierflüssigkeitskonzentrate aufbereitet werden können. Zu diesem Zweck weist das zweite Steckerteil 210 im Buchsenkörper einen weiteren Kanal 300 für die Zuführung von flüssigem Konzentrat auf. Der Kanal führt von der Ausgangsseite des Buchsenkörpers 232 zu dem Hohlraum zwischen der Dichtbuchse 209 und der Ventilbuchse 250, so wie in Fig. 7 und 8 dargestellt. Über eine in den Kanal 300 eingeführte Tülle 302 ist ein Schlauch 304 an den Kanal 300 angeschlossen. Die Tülle 302 ist über einen O-Ring 306 gegen den Buchsenkörper 232 abgedichtet. Der Schlauch 304 ist mit seinem anderen Ende entweder mit einem Konzentratbehälter, beispielsweise für hochkonzentrierte Bicarbonatlösung, oder, beim Spülen des Gerätes, mit dem Dialyslerflüssigkeitskreislauf verbunden. In der in Fig. 7 dargestellten Stellung des zweiten Steckerteils 210 wird Konzentrat aus dem (hier nicht dargestellten) Behälter mit der hochkonzentrierten Bicarbonatlösung in den Hohlraum zwischen der Dichtbuchse 209 und der Ventilbuchse 250 gefördert. Über den Kanal 244 sowie die Querbohrung 213 strömt gegebenenfalls Verdünnungswasser ebenfalls in den Hohlraum der Dichtbuchse 209 und der Ventilbuchse 250. Durch hier nicht dargestellte Steuervorrichtungen wird sichergestellt, daß ein vorbestimmtes Mischungsverhältnis eingehalten wird. Die verdünnte Flüssigkeit wird dann über den Kanal 242 aus dem zweiten Steckerteil 210 heraus dem Dialysiergerät zugeführt.

Bei aufgestecktem erstem Steckerteil 220 wird der Konzentratkanal 300 durch O-Ringe 308, 310, die an dem Ventilelement 250, zweckmäßigerweise am dritten kreisringförmigen Abschnitt 223, angeordnet sind, gegen die Ventilkammer 226 abgedichtet. Die O-Ringe 308, 310 sind in der Weise am dritten kreisringförmigen Abschnitt 223 angeordnet, daß sie bei aufgestecktem Beutel 200 beidseitig der Mündung des Konzentratkanals 300 aufliegen.

Bei dieser Nutzungsweise des Zweiten Steckerteils 210 wird die Reinigung bwz. das Sterilisieren der Kupplungsbuchse in der Weise durchgeführt, daß das andere Ende des Schlauchs 304 vom Kanister mit der hochkonzentrierten Flüssigkeitslösung entfernt und so an das Dialysiergerät angeschlossen wird, daß sowohl durch den Zuführkanal 244 als auch durch den Konzentratkanal 300 Wasser bzw. Desinfektionslösung in das zweite Steckerteil einströmt, diese durchspült und anschließend aus dem Kanal 242 abgeführt wird.

An der Innenfläche 218 des zweiten Steckerteils können korrespondierende Markierungen (hier nicht dargestellt) zum Identifizieren verschiedener Konzentratbeutel vorgesehen sein. Diese Markierungen können z.B. aus Nut- und Federanordnungen oder Paßringen bestehen. Sie stellen sicher, daß nur Beutel mit vorbestimmten Konzentraten an den dafür vorgesehenen Konnektor- bzw. Leitungsanschluß angekoppelt werden können.

## Patentansprüche

1. Beutel, der ein flüssiges oder festes Konzentrat zur Herstellung einer Dialysierflüssigkeit enthält und eine Öffnung aufweist, an der ein die Öffnung dicht umgebender Steckerteil (20, 220) vorgesehen ist, der mit dem Beutelinneren in Strömungsverbindung steht, dadurch gekennzeichnet, daß der Steckerteil (20, 220) Kupplungsmittel zum Verbinden mit einem direkt an einer Dialysemaschine vorgesehenen, komplementären Steckerteil (10, 210) aufweist und daß der Steckerteil doppellumig und mit einem mit dem einen Lumen in Verbindung stehenden Einlaß (82) für aus der Dialysemaschine strömende Flüssigkeit und mit einem mit dem anderen Lumen in Verbindung stehenden Auslaß (90) für verdünntes, zurück zur Dialysemaschine strömendes Konzentrat derart ausgebildet ist, daß ein erster Strömungspfad (96) von der Dialysemaschine zum Beutelinneren und ein zweiter Strömungspfad (98) vom Beutelinneren zurück zur Dialysemaschine ohne Schlauchverbindungen herstellbar ist.

2. Beutel nach Anspruch 1, dadurch gekennzeichnet, daß das den Auslaß (90) aufweisende Lumen des mit dem Beutelinneren in Strömungsverbindung stehenden Steckerteils (20, 220) mit einem in das Beutelinnere ragenden Schlauch verbunden ist.

3. Beutel nach Anspruch 2, dadurch gekennzeichnet, daß der in das Beutelinnere ragende Schlauch an seinem freien Ende mit einem Filter (102) versehen ist.

4. Beutel nach Anspruch 2 oder 3, dadurch gekennzeichnet, daß der erste und zweite Strömungspfad (96, 98) parallel, insbesondere konzentrisch angeordnet sind.

5. Beutel nach einem der Ansprüche 1 bis 4, dadurch gekennzeichnet, daß der mit dem Beutelinneren in Strömungsverbindung stehende Steckerteil (20, 220) eine Codierung zum Kennzeichnen eines bestimmten Beutelinhaltes aufweist.

6. Beutel nach einem der Ansprüche 1 bis 5, dadurch gekennzeichnet, daß der mit dem Beutelinneren in Strömungsverbindung stehende Steckerteil (20, 220) Rastmittel (74, 222) zum Herstellen einer flüssigkeitsdichten Verbindung mit dem komplementären Steckerteil (10, 210) aufweist.

## Claims

**1.** A bag containing a liquid or solid concentrate for the production of a dialysing fluid and which has an opening at which there is a push-in part (20, 220) which surrounds the opening in sealing-tight manner and which is connected to the interior of the bag to allow through-flow, characterised in that the push-in part (20, 220) comprises coupling means for connection to a matching push-in part (10, 210) provided directly on a dialysis machine and in that the push-in part is of double-lumen construction, with an inlet (82) communicating with one lumen and for the fluid flowing from the dialysis machine and with an outlet (90) communicating with the other lumen and for dilute concentrate which flows back to the dialysis machine so that a first flow path (96) can be established from the dialysis machine to the bag interior and a second flow path (98) can be established from the bag interior back to the dialysis machine with no hose connections.

**2.** A bag according to claim 1, characterised in that the lumen of the push-in part (20, 220) which is in flow-permitting communication with the bag interior, and which comprises the outlet (90), is connected to a tube projecting into the bag interior.

**2.** A bag according to claim 2, characterised in that the tube protruding into the bag interior has a filter (102) at its free end.

**4.** A bag according to claim 2 or 3, characterised in that the first and second flow paths (96, 98) are parallel and are in particular concentric.

**5.** A bag according to one of claims 1 to 4, characterised in that the push-in part (20, 220) which is in flow-permitting communication with the bag interior has a coding to identify a specific bag content.

**6.** A bag according to one of claims 1 to 5, characterised in that the push-in part (20, 220) which is in flow-permitting communication with the bag interior comprises catch means (74, 222) for establishing a fluid-tight connection to the matching push-in part (10, 210).

## Revendications

1. Sac qui contient un concentrat liquide ou solide pour la préparation d'un liquide de dialyse et qui présente une ouverture contre laquelle est prévu un élément de connexion (20, 220) entourant l'ouverture de manière étanche, qui se trouve en liaison par écoulement avec l'intérieur du sac, caractérisé en ce que l'élément de connexion (20, 220) présente des moyens de couplage pour la liaison avec un élément de connexion complémentaire (10, 210) prévu directement sur une machine de dialyse et en ce que l'élément de connexion est réalisé sous la forme d'un élément de connexion à double lumière et comprenant une entrée (82) pour du liquide s'écoulant hors de la machine de dialyse, se trouvant en liaison avec la première lumière et une sortie (90) pour du concentrat dilué s'écoulant en retour en direction de la machine de dialyse, se trouvant en liaison avec l'autre lumière, de telle sorte que l'on puisse réaliser une première voie d'écoulement (96) depuis la machine de dialyse jusqu'à l'intérieur du sac et une seconde voie d'écoulement (98) depuis l'intérieur du sac en retour jusqu'à la machine de dialyse en l'absence de liaisons sous forme de tuyaux flexibles.

2. Sac selon la revendication 1, caractérisé en ce que la lumière présentant la sortie (90), de l'élément de connexion (20, 220) se trouvant en liaison par écoulement avec l'intérieur du sac est reliée à un tuyau flexible faisant saillie à l'intérieur du sac.

3. Sac selon la revendication 2, caractérisé en ce que le tuyau flexible faisant saillie à l'intérieur du sac est muni d'un filtre (102) à son extrémité libre.

4. Sac selon la revendication 2 ou 3, caractérisé en ce que les première et deuxième voies d'écoulement (96, 98) sont disposées en parallèle, en particulier en position concentrique.

5. Sac selon l'une quelconque des revendications 1 à 4, caractérisé en ce que l'élément de connexion (20, 220) qui se trouve en liaison par écoulement avec l'intérieur du sac présente un code pour répertorier un contenu de sac déterminé.

6. Sac selon l'une quelconque des revendications 1 à 5, caractérisé en ce que l'élément de connexion (20, 220) qui se trouve en liaison par écoulement avec l'intérieur du sac présente des agents d'encliquetage (74, 222) pour réaliser une liaison étanche aux fluides avec l'élément de connexion complémentaire (10, 210).
